(19) Europäisches Patentamt
European Patent Office
Office européen des brevets

(11) Numéro de publication : **0 109 866**
**B1**

(12) # FASCICULE DE BREVET EUROPÉEN

(45) Date de publication du fascicule du brevet : **19.08.87**

(21) Numéro de dépôt : **83401824.4**

(22) Date de dépôt : **20.09.83**

(51) Int. Cl.⁴ : **C 07 D209/46**, C 07 D401/04, C 07 D405/04, C 07 D409/04, A 61 K 31/64

(54) **Nouveaux dérivés de la sulfonylurée, leurs procédés de préparation et les compositions pharmaceutiques les renfermant.**

(30) Priorité : **23.09.82 FR 8216012**

(43) Date de publication de la demande : **30.05.84 Bulletin 84/22**

(45) Mention de la délivrance du brevet : **19.08.87 Bulletin 87/34**

(84) Etats contractants désignés : **AT BE CH DE FR GB IT LI LU NL SE**

(56) Documents cités :
**DE-A- 1 670 660**
**FR-M- 4 852**

(73) Titulaire : **ADIR**
**22, rue Garnier**
**F-92200 Neuilly-sur-Seine (FR)**

(72) Inventeur : **Beregi, Laszlo**
**257 Bld. Jean-Jaurès**
**F-92100 Boulogne-Billancourt (FR)**
Inventeur : **Hugon, Pierre**
**68 rue Voltaire**
**F-92500 Rueil Malmaison (FR)**
Inventeur : **Duhault, Jacques**
**14bis rue Paul Demange**
**F-78290 Croissy-sur-Seine (FR)**
Inventeur : **Boulanger, Michelle**
**71 Av. Auguste Renoir**
**F-78160 Marly-le-Roi (FR)**

(74) Mandataire : **Reverbori, Marcelle**
**ADIR 22 Rue Garnier**
**F-92200 Neuilly-sur-Seine (FR)**

**EP 0 109 866 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux dérivés de la sulfonylurée, leurs procédés de préparation et les compositions pharmaceutiques les renfermant.

Elle concerne particulièrement les dérivés de la sulfonylurée de formule générale I :

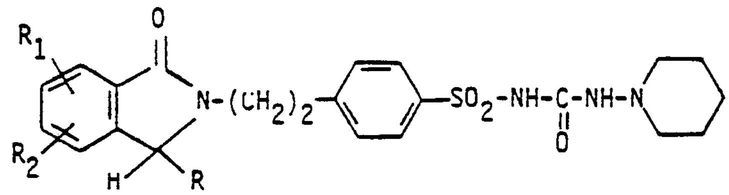

(I)

dans laquelle :

R représente un radical thiényle ou un radical phényle éventuellement substitué par un atome d'halogène ou un radical trifluorométhyle ;

$R_1$ et $R_2$ identiques ou différents représentent chacun un atome d'hydrogène ou d'halogène, ou un radical alcoxy contenant de 1 à 5 atomes de carbone, ou forment ensemble le groupe méthylène dioxy ($-O-CH_2-O$).

L'état antérieur de la technique dans le domaine des sulfonylurées hypoglycémiantes est illustré principalement par les brevets DE-A-1 670 660 et Fr.M.4852, lesquels concernent des dérivés de formule générale

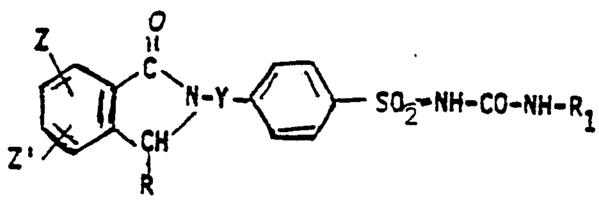

dans laquelle

R est hydrogène ou alkyle inférieur

Z et Z' sont hydrogène, halogène ou alkyle ou alkoxy inférieurs

Y est un groupe hydrocarboné en $C_1$ ou $C_2$, et

$R_1$ représente, entre autres, un groupe alkyle, alcényle, cycloalkyle ou cycloalcényle mais ne représente jamais un radical hétérocyclique azoté comme c'est le cas pour les dérivés de la présente demande qui sont des pipéridino sulfonylurées.

La présente invention a également pour objet le procédé de préparation des dérivés de formule générale I caractérisé en ce que l'on traite par $SOCl_2$ les acides benzoïques de formule générale II :

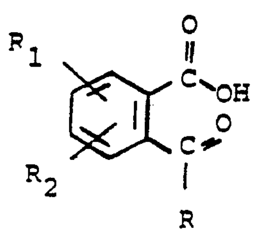

(II)

dans laquelle R, $R_1$ et $R_2$ ont les significations précédemment définies, en vue d'obtenir les isobenzofuranones de formule générale III :

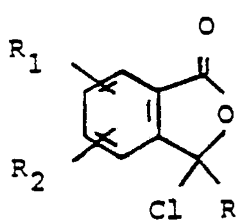

(III)

que l'on condense avec le composé de formule générale IV :

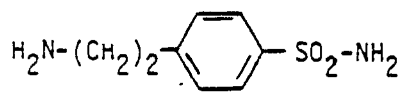

(IV)

pour obtenir les 3-hydroxy iso-indolones de formule générale V :

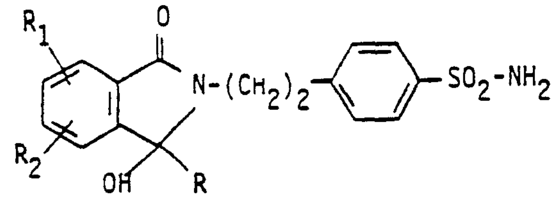

(V)

dans laquelle R, $R_1$ et $R_2$ sont tels que précédemment définis, lesquels composés (V) sont transformés en isoindolones de formule générale VI :

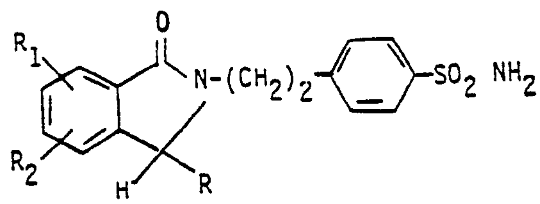

(VI)

dans laquelle R, $R_1$ et $R_2$ sont tels que définis précédemment, et l'on condense ces isoindolones (VI) avec un composé de formule VII :

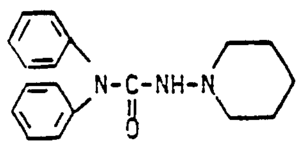

(VII)

La présente invention comprend également les sels formés avec les hydroxydes alcalins ou alcalino-terreux, les carbonates alcalins ou alcalino-terreux et les bicarbonates alcalins.

Les dérivés de formule générale I et leurs sels physiologiquement tolérables possèdent des propriétés thérapeutiques intéressantes en particulier une activité hypoglycémiante remarquable. Ils peuvent donc être utilisés comme médicaments actifs par voie orale dans le traitement du diabète. Leur toxicité est très faible et la $DL_{50}$ étudiée chez la souris est supérieure à 3 g/kg per os pour tous les dérivés.

L'activité hypoglycémiante a été étudiée chez le lapin et le rat par voie orale. La dose minima active se situe entre 1 et 10 mg/kg et on obtient une baisse de 30 % de la glycémie avec des doses variant entre 1 et 25 mg/kg avec la plupart des dérivés.

A titre de comparaison, le chloropropamide, hypoglycémiant bien connu, doit être administré à la dose de 50 mg/kg chez le rat pour obtenir le même effet. D'autre part, la $DL_{50}$ de ce produit est de 1 g/kg ; ce produit est donc 3 fois plus toxique que les dérivés de la présente invention.

Ces nouveaux dérivés peuvent être administrés, aux malades atteints de diabète, sous différentes forme pharmaceutiques, de préférence sous forme de comprimés, dragées, capsules ou gélules pour administration par voie orale, en association avec les supports pharmaceutiques appropriés comme, par exemple, le talc, l'amidon, le lactose ou le stéarate de magnésium. Les doses utilisées peuvent varier de 2,5 à 100 mg et de préférence de 5 à 30 mg par jour.

La présente invention a également pour objet les compositions pharmaceutiques contenant comme principe actif un dérivé de formule générale I ou un de ses sels physiologiquement tolérables, mélangé ou associé à un excipient pharmaceutique approprié.

Les exemples suivants donnés à titre non-limitatifs illustrent l'invention. Toutes les parties sont exprimées en poids et les points de fusion sont déterminés par la méthode de Kofler.

Exemple 1

3-chloro 3-phényl 1-(3H) isobenzofuranone

22,5 parties d'acide 2-benzoyl benzoïque sont ajoutées par petites portions, en 15 minutes sous agitation à 40 parties de chlorure de thionyle. Le mélange réactionnel est ensuite porté lentement à reflux et celui-ci est maintenu pendant 90 minutes. Le chlorure de thionyle en excès est éliminé sous vide et le

résidu est repris par 50 parties de benzène anhydre puis évaporé à sec sous vide. L'opération est recommencée une autre fois puis le résidu est repris par 80 parties de tétrahydrofuranne anhydre. Cette solution est utilisée telle quelle dans la phase suivante

Exemples 2 à 10

Les dérivés suivants ont été préparés selon la méthode décrite dans l'exemple 1 :

2) 3-chloro 3-parafluorophényl 1-(3H) isobenzofuranone, à partir de l'acide 2-parafluorobenzoyl benzoïque.

3) 3-chloro 3-phényl 5-bromo 1-(3H) isobenzofuranone, à partir de l'acide 2-benzoyl 4-bromobenzoïque.

4) 3-chloro 3-phényl 5,6-diméthoxy 1-(3H) isobenzofuranone, à partir de l'acide 2-benzoyl 4,5-diméthoxybenzoïque.

5) 3-chloro 3-(2-thiényl) 1-(3H) isobenzofuranone, à partir de l'acide 2-(2-thiénoyl) benzoïque.

6) 3-chloro 3-phényl 5-méthoxy 1-(3H) isobenzofuranone, à partir de l'acide 2-benzoyl 4-méthoxybenzoïque.

7) 3-chloro 3-phényl 5,6-méthylènedioxy 1-(3H) isobenzofuranone, à partir de l'acide 2-benzoyl 4,5-méthylènedioxy benzoïque.

8) 3-chloro 3-métatrifluorométhylphényl 1-(3H) isobenzofuranone, à partir de l'acide 2-métatrifluorométhyl benzoyl benzoïque.

9) 3-chloro 3-métafluorophényl 1-(3H) isobenzofuranone, à partir de l'acide 2-métafluorobenzoyl benzoïque.

10) 3-chloro 3-orthofluorophényl 1-(3H) isobenzofuranone, à partir de l'acide 2-orthofluorobenzoyl benzoïque.

Exemple 11

para [β-(2,3-dihydro 3-hydroxy 3-phényl (1H) iso-indol-1-one 2-yl) éthyl] benzènesulfonamide.

A une solution de 10 parties de para (β-amino éthyl) benzènesulfonamide et 5,06 parties de triéthylamine dans 80 parties de diméthylformamide anhydre on ajoute sous agitation et en 15 minutes environ, 12,35 parties de 3-chloro 3-phényl 1-(3H) isobenzofuranone en solution dans 40 parties de tétrahydrofuranne anhydre. La température s'élève de 25 à 50 °C et après 2 heures d'agitation, le mélange réactionnel est dilué dans 400 parties d'eau. Le précipité formé est essoré et séché à l'air puis recristallisé dans 360 parties d'isopropanol. On obtient 12 parties de para-(2,3-dihydro 3-hydroxy 3-phényl (1H) iso-indol-1-one 2-yl) éthyl benzènesulfonamide fondant à 225 °C.

Exemples 12 à 20

Les dérivés suivants ont été préparés selon la méthode décrite dans l'exemple 11 :

12) para [β-(2,3-dihydro 3-hydroxy 3-parafluorophényl (1H) iso-indol-1-one 2-yl) éthyl] benzènesulfonamide, P.F. : 231-232 °C (isopropanol), à partir de 3-chloro 3-parafluorophényl 1-(3H) isobenzofuranone et de para β-amino éthyl) benzènesulfonamide.

13) para [β-(2,3-dihydro 3-hydroxy 3-phényl 5-bromo (1H) isoindol-1-one 2-yl) éthyl] benzènesulfonamide, P.F. 248-250 °C (éthanol), à partir de 3-chloro 3-phényl 5-bromo 1-(3H) isobenzofuranone et de para (β-amino éthyl) benzènesulfonamide ;

14) para [β-(2,3-dihydro 3-hydroxy 5,6-diméthoxy (1H) iso-indol-1-one 2-yl) éthyl] benzènesulfonamide, P.F. : 225 °C (acétonitrile), à partir de 3-chloro 3-phényl 5,6-diméthoxy 1-(3H) isobenzofuranone et de para (β-amino éthyl) benzènesulfonamide.

15) para [β-(2,3-dihydro 3-hydroxy 3-(2-thiényl) (1H) iso-indol-1-one 2-yl) éthyl] benzènesulfonamide, P.F. : 188 °C (acétonitrile), à partir de 3-chloro 3-(2-thiényl) 1-(3H) isobenzofuranone et de para (β-amino éthyl) benzènesulfonamide.

16) para [β-(2,3-dihydro 3-hydroxy 3-phényl 5-méthoxy (1H) iso-indol-1-one 2-yl) éthyl] benzènesulfonamide P.F. : 193-194 °C (isopropanol), à partir de 3-chloro 3-phényl 5-méthoxy 1-(3H) isobenzofuranone et de para (β-amino éthyl) benzènesulfonamide.

17) para [β-(2,3-dihydro 3-hydroxy 3-phényl 5,6-méthylènedioxy (1H) iso-indol-1-one 2-yl) éthyl] benzènesulfonamide, P.F. : 216-218 °C (éthanol), à partir de la 3-chloro 3-phényl 5,6-méthylènedioxy 1-(3H) isobenzofuranone et de para (β-amino éthyl) benzènesulfonamide.

18) para [β-(2,3-dihydro 3-hydroxy 3-métafluorophényl (1H) iso-indol-1-one 2-yl) éthyl] benzenèsulfonamide P.F. : 234-236 °C (éthanol), à partir de la 3-chloro 3-métafluorophényl 1-(3H) isobenzofuranone et de para (β-amino éthyl) benzènesulfonamide.

19) para [β-(2,3-dihydro 3-hydroxy 3-(méta-trifluorométhylphényl) (1H) iso-indol-1-one 2-yl) éthyl] benzènesulfonamide, P.F. : 230 °C (isopropanol), à partir de la 3-chloro 3-métatrifluorométhylphényl 1-(3H) isobenzofuranone et de para (β-amino éthyl) benzènesulfonamide.

20) para [β-(2,3-dihydro 3-hydroxy 3-orthofluorophényl (1H) iso-indol-1-one 2-yl) éthyl] benzènesulfonamide P.F. : 210 °C (isopropanol), à partir de la 3-chloro 3-orthofluorophényl 1-(3H) isobenzofuranone

et de para (β-amino éthyl) benzènesulfonamide.

Exemple 21

para [β-(2,3-dihydro 3-phényl (1H) iso-indol-1-one, 2-yl) éthyl] benzènesulfonamide

12 parties de para [β-(2,3-dihydro 3-hydroxy 3-phényl (1H) iso-indol-1-one 2-yl) éthyl] benzènesulfonamide sont refluées avec 120 parties d'acide formique à 98 % pendant 7 heures. Après concentration sous vide, le résidu est recristallisé dans 110 parties d'acétate d'éthyle pour donner 8 parties du produit attendu P.F. : 218 °C.

Exemples 22 à 30

Les dérivés suivants ont été préparés selon la méthode décrite dans l'exemple 21 :

22) para [β-(2,3-dihydro 3-parafluorophényl (1H) iso-indol-1-one 2-yl) éthyl] benzènesulfonamide ; P.F. : 192 °C (acétate d'éthyle), à partir de para [β-(2,3-dihydro 3-hydroxy 3-parafluorophényl (1H) iso-indol-1-one, 2-yl) éthyl] benzènesulfonamide.

23) para [β-(2,3-dihydro 3-phényl 5-bromo (1H) iso-indol-1-one 2-yl) éthyl] benzènesulfonamide : P.F. : 245 °C (acétonitrile), à partir de para [β-(2,3-dihydro, 3-hydroxy 3-phényl 5-bromo (1H) iso-indol-1-one 2-yl) éthyl] benzènesulfonamide.

24) para [β-(2,3-dihydro 3-phényl, 5,6-diméthoxy (1H) iso-indol-1-one 2-yl) éthyl] benzènesulfonamide P.F. : 200 °C (acétonitrile), à partir de para [β-(2,3-dihydro 3-hydroxy 3-phényl 5,6-diméthoxy (1H) iso-indol-1-one 2-yl) éthyl] benzènesulfonamide.

25) para [β-(2,3-dihydro 3-(2-thiényl) (1H) iso-indol-1-one 2-yl) éthyl] benzènesulfonamide P.F. : 230 °C (acétate d'éthyle), à partir de para [β-(2,3-dihydro 3-hydroxy 3-(2-thiényl) (1H) iso-indol-1-one 2-yl) éthyl] benzènesulfonamide.

26) para [β-(2,3-dihydro 3-phényl 5-méthoxy (1H) iso-indol-1-one 2-yl) éthyl] benzènesulfonamide P.F. : 204-205 °C (isopropanol), à partir de para [β-(2,3-dihydro 3-hydroxy 3-phényl 5-méthoxy (1H) iso-indol-1-one 2-yl) éthyl] benzènesulfonamide.

27) para [β-(2,3-dihydro 3-phényl 5,6-méthylènedioxy (1H) iso-indol-1-one 2-yl) éthyl] benzènesulfonamide P.F. : 229-230 °C (DMF-$H_2O$), à partir de para [β-(2,3-dihydro 3-hydroxy 3-phényl 5,6-méthylènedioxy (1H) iso-indol-1-one, 2-yl) éthyl] benzènesulfonamide.

28) para [β-(2,3-dihydro 3-métafluorophényl (1H) iso-indol-1-one 2-yl) éthyl] benzènesulfonamide P.F. : 224 °C, (acétonitrile), à partir de para [β-(2,3-dihydro 3-hydroxy 3-(métafluorophényl) (1H) iso-indol-1-one 2-yl) éthyl] benzènesulfonamide.

29) para [β-(2,3-dihydro 3-métatrifluorométhylphényl, (1H) iso-indol-1-one 2-yl) éthyl] benzènesulfonamide P.F. : 204 °C (acétate d'éthyle), à partir de para [β-(2,3-dihydro 3-hydroxy 3-(métatrifluorométhylphényl) (1H) iso-indol-1-one 2-yl) éthyl] benzènesulfonamide.

30) para [β-(2,3-dihydro 3-(orthofluorophényl) (1H) iso-indol-1-one 2-yl) éthyl] benzènesulfonamide P.F. : 220 °C (isopropanol), à partir de para [β-(2,3-dihydro 3-hydroxy 3-(orthofluorophényl) (1H) iso-indol-1-one 2-yl) éthyl] benzènesulfonamide.

Exemple 31

1-{para [β-(2,3-dihydro 3-phényl (1H) iso-indol-1-one 2-yl) éthyl] benzènesulfonyl} 3-pipéridino-urée :

A 8 parties de para [β-(2,3-dihydro 3-phényl (1H) iso-indol-1-one 2-yl) éthyl] benzènesulfonamide sous forme de sel de sodium, en suspension dans 50 parties de diméthylformamide sont ajoutées 5,6 parties de 1,1-diphényl 3-pipéridino-urée. Le mélange réactionnel est ensuite porté sous agitation à 90-95 °C. A cette température, il y a solubilisation puis, après 10 minutes, il se forme un nouveau précipité. Après une heure de chauffage et après refroidissement, le précipité est essoré, lavé à l'éther et séché sous vide. On obtient 6,2 parties du produit attendu sous forme de sel de sodium, P.F. : supérieur à 260 °C.

Exemples 32 à 40

Les dérivés suivants ont été préparés selon la méthode décrite dans l'exemple 31 :

32) 1-{para [β-(2,3-dihydro 3-phényl 5-bromo (1H) iso-indol-1-one 2-yl) éthyl] benzènesulfonyl} 3-piperidino-urée : P.F. : 220-222 °C (diméthylformamide/$H_2O$), à partir de para [β-(2,3-dihydro 3-phényl 5-bromo (1H) iso-indol-1-one 2-yl) éthyl] benzènesulfonamide et de la 1,1-diphényl 3-pipéridino-urée.

33) 1-{para [β-(2,3-dihydro 3-phényl 5,6-diméthoxy (1H) iso-indol-1-one 2-yl) éthyl] benzènesulfonyl} 3-pipéridino-urée, isolée sous forme de son sel de sodium P.F. : supérieur à 260 °C, à partir de para [β-(2,3-dihydro 3-phényl 5,6-diméthoxy (1H) iso-indol-1-one 2-yl) éthyl] benzènesulfonamide et de la 1,1-diphényl 3-piperidino-urée.

34) 1-{para [b-(2,3-dihydro 3-(2-thiényl) (1H) iso-indol-1-one 2-yl) éthyl] benzènesulfonyl} 3-pipéridino-urée : P.F. : 150 °C (acétonitrile), à partir de para [β-(2,3-dihydro 3-(2-thiényl) (1H) iso-indol-1-one 2-yl) éthyl] benzènesulfonamide et de la 1,1-diphényl 3-pipéridino-urée.

35) 1-{para [β-(2,3-dihydro, 3-parafluorophényl (1H) iso-indol-1-one 2-yl) éthyl] benzènesulfonyl} 3-pipéridino-urée P.F. : 190-191 °C (isopropanol), à partir de para [β-(2,3-dihydro 3-parafluorophenyl (1H) iso-indol-1-one 2-yl) éthyl] benzènesulfonamide et de la 11-diphényl 3-pipéridino-urée.

36) 1-{para [β-(2,3-dihydro 3-phényl 5-méthoxy (1H) iso-indol-1-one 2-yl) éthyl] benzènesulfonyl} 3-pipéridino-urée, isolées sous forme de son sel de sodium P.F. : supérieur à 260 °C, à partir de para [β-(2,3-dihydro 3-phényl 5-méthoxy (1H) iso-indol-1-one 2-yl) éthyl] benzènesulfonamide et de la 1,1-diphényl 3-pipéridino-urée.

37) 1-{para [β-(2,3-dihydro 3-phényl 5,6-méthylènedioxy (1H) iso-indol-1-one 2-yl) éthyl] benzènesulfonyl} 3-pipéridino-urée, isolée sous la forme de son sel de sodium P.F. : supérieur à 260 °C, à partir de para [β-(2,3-dihydro 3-phényl 5,6-méthylènedioxy (1H) iso-indol-1-one 2-yl) éthyl] benzènesulfonamide et de la 1,1-diphényl 3-pipéridino-urée.

38) 1-{para [β-(2,3-dihydro 3-métatrifluorométhylphényl (1H) iso-indol-1-one 2-yl) éthyl] benzènesulfonyl} 3-pipéridino-urée, isolée sous la forme de son sel de sodium, P.F. : supérieur à 260 °C, à partir de para [β-(2,3-dihydro 3-métatrifluorométhylphényl (1H) iso-indol-1-one 2-yl) éthyl] benzènesulfonamide et de la 1,1-diphényl 3-pipéridino-urée.

39) 1-{para [β-(2,3-dihydro 3-métafluorophényl (1H) iso-indol-1-one 2-yl) éthyl] benzènesulfonyl} 3-pipéridino-urée, isolée sous la forme de son sel de sodium, P.F. : supérieur à 260 °C, à partir de para [β-(2,3-dihydro 3-métafluorophényl (1H) iso-indol-1-one 2-yl) éthyl] benzènesulfonamide et de la 1,1-diphényl 3-pipéridino-urée.

40) 1-{para [β-(2,3-dihydro 3-orthofluorophényl (1H) iso-indol-1-one 2-yl) éthyl] benzènesulfonyl} 3-pipéridino-urée, isolée sous la forme de son sel de sodium, P.F. : supérieur à 260 °C, à partir de para [β-(2,3-dihydro 3-orthofluorophényl (1H) iso-indol-1-one 2-yl) éthyl] benzènesulfonamide et de la 1,1-diphényl 3-pipéridino-urée.

**Revendications** (pour les Etats contractants : BE, CH, DE, FR, GB, IT, LI, LU, NL SE)

1. Les dérivés de la sulfonylurée de formule générale I :

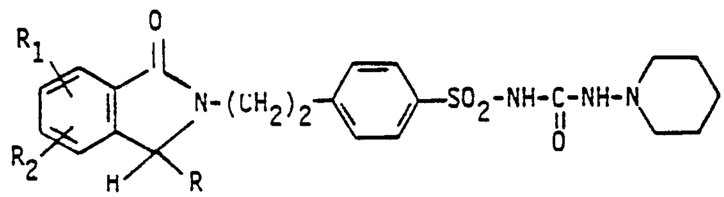

(I)

dans laquelle

R représente un radical thiényle ou un radical phényle éventuellement substitué par un atome d'halogène ou radical trifluorométhyle ;

$R_1$ et $R_2$ identiques ou différents représentent chacun un atome d'hydrogène ou d'halogène, ou un radical alcoxy contenant de 1 à 5 atomes de carbone, ou forment ensemble le groupe méthylène dioxy ($-O-CH_2-O-$).

2. Les sels des composés de la revendication 1 avec des agents basiques appropriés.

3. Les sels selon la revendication 2 qui sont physiologiquement tolérables.

4. La 1-{para [β-(2,3-dihydro 3-phényl (1H) iso-indol-1-one 2-yl) éthyl] benzènesulfonyl} 3-pipéridino-urée et son sel de sodium.

5. La 1-{para [β-(2,3-dihydro 3-phényl 5,6-méthylènedioxy (1H) iso-indol-1-one 2-yl)] éthyl bensènesulfonyl} 3-pipéridino-urée et son sel de sodium.

6. Un procédé de préparation des composés selon la revendication 1, caractérisé en ce que : l'on traite par $SOCl_2$ les acides benzoïques de formule générale II :

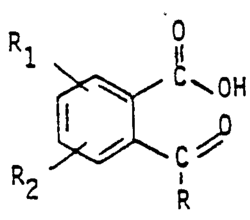

(II)

dans laquelle R, $R_1$ et $R_2$ ont les significations définies dans la revendication 1, en vue d'obtenir les isobenzofuranones de formule générale III :

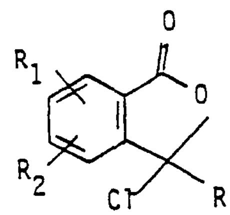

(III)

que l'on condense avec le composé de formule générale IV :

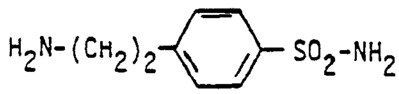

(IV)

pour obtenir les 3-hydroxy iso-indolones de formule générale V :

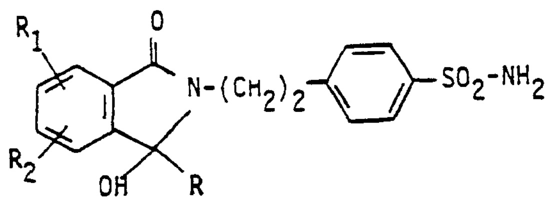

(V)

dans laquelle R, $R_1$ et $R_2$ sont tels que définis dans la revendication 1, lesquels composés V sont transformés en isoindolones de formule générale VI :

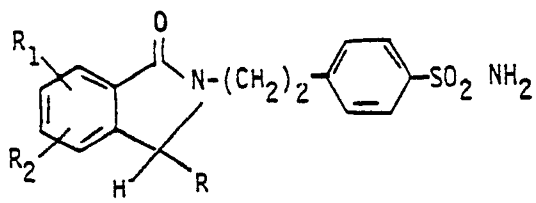

(VI)

dans laquelle R, $R_1$ et $R_2$ sont tels que définis dans la revendication 1 et l'on condense ces isoindolones (VI) avec un composé de formule VII :

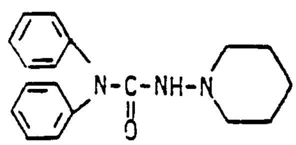

(VII)

7. Les compositions pharmaceutiques contenant comme principe actif un composé selon les revendications 1 ou 3 à 5, avec les excipients pharmaceutiques appropriés.

8. Les compositions pharmaceutiques selon la revendication 7 présentées sous une forme convenant notamment pour le traitement du diabète.

**Revendication** (pour l'Etat contractant AT)

Procédé de préparation des dérivés de la sulfonylurée de formule générale I :

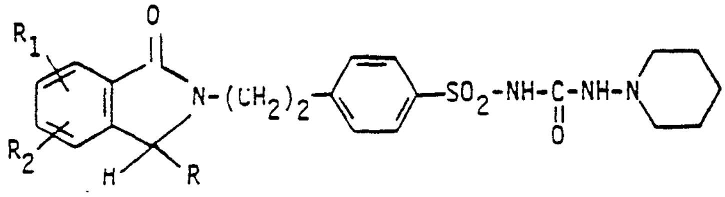

(I)

dans laquelle

R représente un radical thiényle ou un radical phényle éventuellement substitué par un atome d'halogène ou radical trifluorométhyle ;

$R_1$ et $R_2$ identiques ou différents représentent chacun un atome d'hydrogène ou d'halogène, ou un radical alcoxy contenant de 1 à 5 atomes de carbone, ou forment ensemble le groupe méthylène dioxy (—O—$CH_2$—O—),

et de leurs sels d'addition avec des agents basiques appropriés caractérisé en ce que : l'on traite par $SOCl_2$ les acides benzoiques de formule générale II :

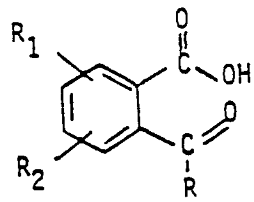

(II)

dans laquelle R, $R_1$ et $R_2$ ont les significations définies ci-dessus, en vue d'obtenir les isobenzofuranones de formule générale III :

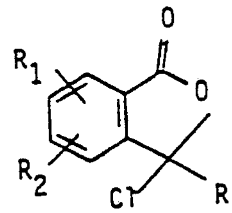

(III)

que l'on condense avec le composé de formule IV :

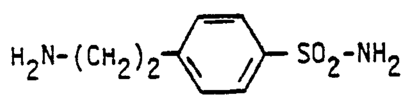

(IV)

pour obtenir les 3-hydroxy iso-indolones de formule générale V :

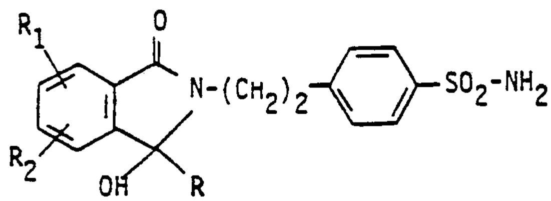

(V)

dans laquelle R, $R_1$ et $R_2$ sont tels que définis précédemment, lesquels composés V sont transformés en isoindolones de formule générale VI :

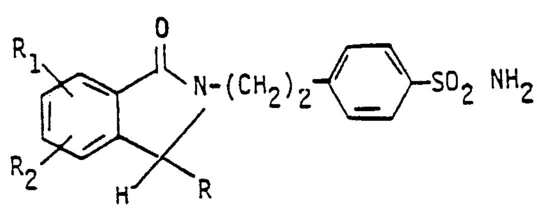

(VI)

dans laquelle R, $R_1$ et $R_2$ sont tels que définis précédemment et l'on condense ces isoindolones (VI) avec un composé de formule VII :

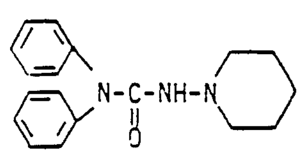

(VII)

**Claims** (for the Contracting States : BE, CH, DE, FR, GB, IT, LU, NL, SE)

1. Sulphonylurea derivatives of the general formula I :

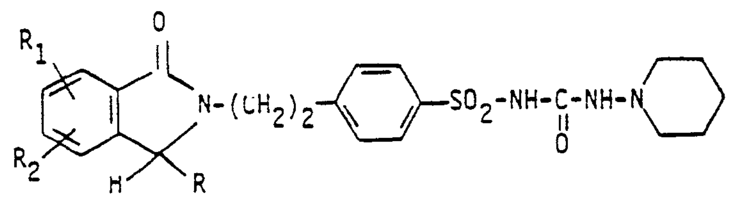

(I)

in which

R represents a thienyl radical or a phenyl radical optionally substituted by a halogen atom or by a trifluoromethyl radical ;

$R_1$ and $R_2$ are the same or different and each represents a hydrogen atom or a halogen atom, or an alkoxy radical containing from 1 to 5 carbon atoms, or they together form the methylenedioxy group ($-O-CH_2-O-$).

2. The salts of the compounds of claim 1 with suitable basic agents.

3. The salts according to claim 2 that are physiologically tolerable.

4. 1-{para-[β-(2,3-dihydro-3-phenyl-(1H)-isoindol-1-on-2-yl) ethyl] benzenesulphonyl}-3-piperidinourea and the sodium salt thereof.

5. 1-{para-[β-(2,3-dihydro-3-phenyl-5,6-methylenedioxy-(1H)-isoindol-1-on-2-yl) ethyl] benzenesulphonyl}-3-piperidinourea and the sodium salt thereof.

6. A process for the preparation of the compounds according to claim 1, characterized in that : the benzoic acids of the general formula II :

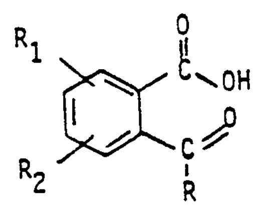

(II)

in which R, $R_1$ and $R_2$ have the meanings defined in claim 1, are treated with $SOCl_2$ in order to obtain the isobenzofuranones of the general formula III :

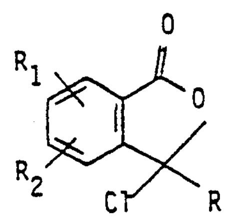

(III)

which are condensed with the compound of the general formula IV :

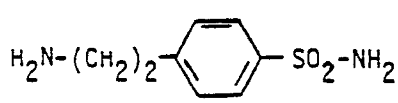

(IV)

to obtain the 3-hydroxyisoindolones of the general formula V :

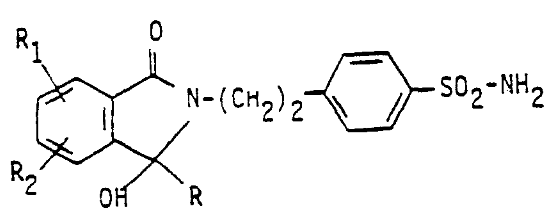

(V)

in which R, $R_1$ and $R_2$ are as defined in claim 1, which compounds V are converted into isoindolones of the general formula VI :

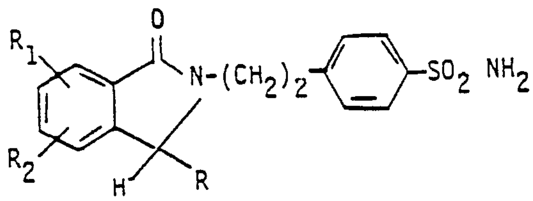

(VI)

in which R, $R_1$ and $R_2$ are as defined in claim 1, and these isoindolones (VI) are condensed with a compound of the formula VII :

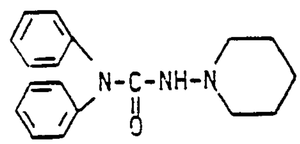

(VII)

7. Pharmaceutical compositions containing, as active ingredient, a compound according to claims 1 or 3 to 5 together with suitable pharmaceutical excipients.

8. Pharmaceutical compositions according to claim 7 presented in a form suitable, especially, for the treatment of diabetes.

**Claim** (for the Contracting State AT)

Process for the preparation of sulphonylurea derivatives of the general formula I :

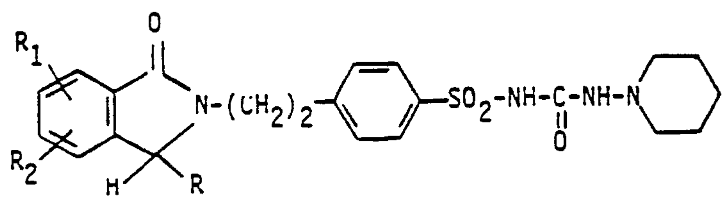

(I)

in which

R represents a thienyl radical or a phenyl radical optionally substituted by a halogen atom or by a trifluoromethyl radical ;

$R_1$ and $R_2$ are the same or different and each represents a hydrogen atom or a halogen atom, or an alkoxy radical containing from 1 to 5 carbon atoms, or they together form the methylenedioxy group ($-O-CH_2-O-$)

and their addition salts with suitable basic agents, characterised in that : the benzoic acids of the general formula II :

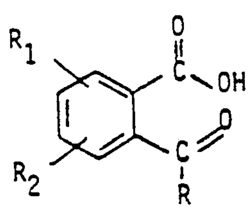

(II)

in which R, $R_1$ and $R_2$ have the meanings defined above are treated with $SOCl_2$ in order to obtain the isobenzofuranones of the general formula III :

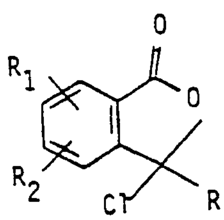

(III)

which are condensed with the compound of the formula IV :

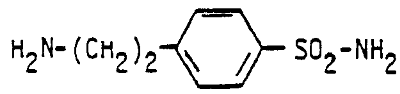

(IV)

to obtain the 3-hydroxyisoindolones of the general formula V :

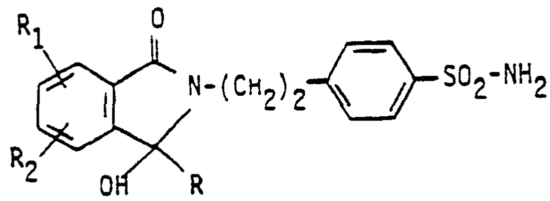

(V)

in which R, $R_1$ and $R_2$ are as defined above, which compounds V are converted into isoindolones of the general formula VI :

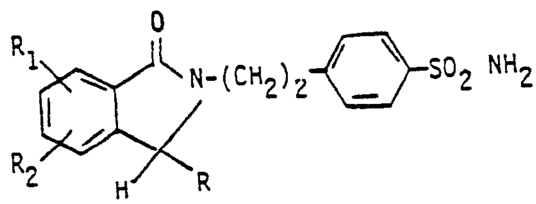

(VI)

in which R, $R_1$ and $R_2$ are as defined above, and these isoindolones (VI) are condensed with a compound of the formula VII :

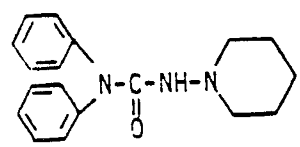

(VII)

**Patentansprüche** (für die Vertragsstaaten : BE, CH, DE, FR, GB, IT, LU, NL, SE)

1. Sulfonylharnstoffderivate der allgemeinen Formel I

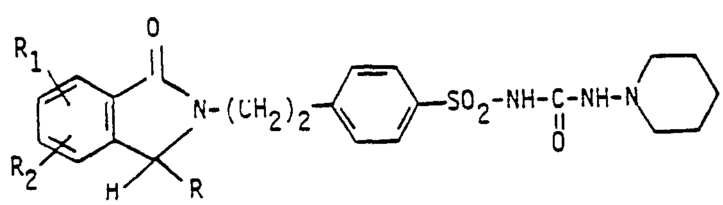

(I)

in der

R einen Thienylrest oder einen Phenylrest, der gegebenenfalls durch ein Halogenatom oder eine Trifluormethylgruppe substituiert ist ; und

$R_1$ und $R_2$, die gleichartig oder verschieden sein können, jeweils Wasserstoffatome, Halogenatome oder Alkoxygruppen mit 1 bis 5 Kohlenstoffatomen oder gemeinsam eine Methylendioxygruppe ($—O—CH_2—O—$) bedeuten.

2. Die Salze der Verbindungen von Anspruch 1 mit geeigneten basischen Mitteln.

3. Die Salze nach Anspruch 2, die physiologisch verträglich sind.

4. 1-{p-[β-(2,3-Dihydro-3-phenyl-(1H)-isoindol-1-on-2-yl)-ethyl]-benzolsulfonyl}-3-piperidino-harnstoff und dessen Natriumsalz.

5. 1-{p-[β-(2,3-Dihydro-3-phenyl-5,6-methylendioxy-(1H)-isoindol-1-on-2-yl)-ethyl]-benzolsulfonyl)-3-piperidino-harnstoff und dessen Natriumsalz.

6. Verfahren zur Herstellung der Verbindungen nach Anspruch 1, dadurch gekennzeichnet, daß man die Benzoesäuren der allgemeinen Formel II

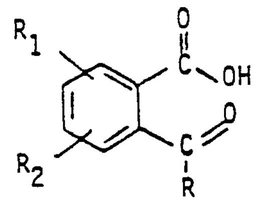

(II)

in der R, $R_1$ und $R_2$ die in Anspruch 1 angegebenen Bedeutungen besitzen, mit $SOCl_2$ zur Bildung von Isobenzofuranonen der allgemeinen Formel III

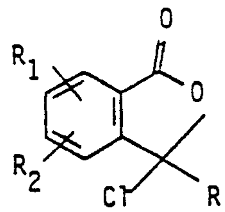

(III)

behandelt, welche man mit der Verbindung der allgemeinen Formel IV

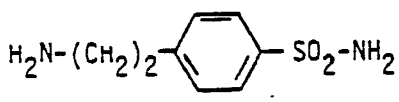

(IV)

zu einem 3-Hydroxyisoindolon der allgemeinen Formel V

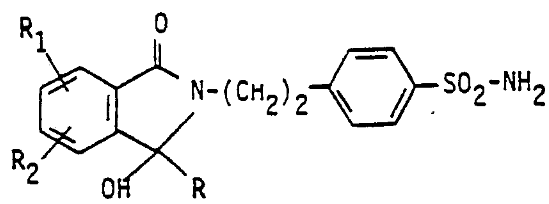

(V)

in der R, $R_1$ und $R_2$ die in Anspruch 1 angegebenen Bedeutungen besitzen, kondensiert, welche Verbindungen der allgemeinen Formel V man in die Isoindolone der allgemeinen Formel VI

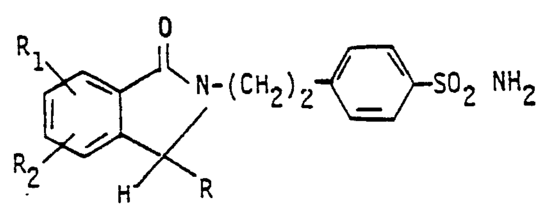

(VI)

in der R, $R_1$ und $R_2$ die in Anspruch 1 angegebenen Bedeutungen besitzen, umwandelt, und man diese Indolone der Formel (VI) mit einer Verbindung der Formel VII

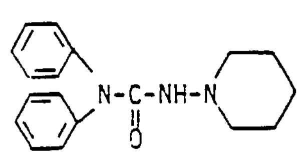

(VII)

kondensiert.

7. Pharmazeutische Zubereitungen enthaltend als Wirkstoff eine Verbindung nach einem der

Ansprüche 1 oder 3 bis 5 zusammen mit geeigneten pharmazeutischen Trägermaterialien.

8. Pharmazeutische Zubereitungen nach Anspruch 7, dadurch gekennzeichnet, daß sie in einer zur Behandlung des Diabetes geeigneten Form vorliegen.

**Patentanspruch** (für den Vertragsstaat AT)

Verfahren zur Herstellung von Sulfonylharnstoffderivaten der allgemeinen Formel I

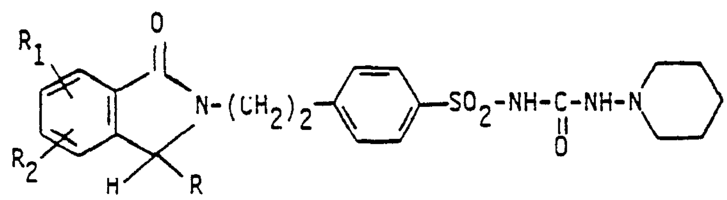

(I)

in der

R einen Thienylrest oder einen Phenylrest, der gegebenenfalls durch ein Halogenatom oder eine Trifluormethylgruppe substituiert ist ; und

$R_1$ und $R_2$, die gleichartig oder verschieden sein können, jeweils Wasserstoffatome, Halogenatome oder Alkoxygruppen mit 1 bis 5 Kohlenstoffatomen oder gemeinsam eine Methylendioxygruppe ($-O-CH_2-O-$) bedeuten,

und der Additionssalze mit geeigneten basischen Mitteln, dadurch gekennzeichnet, daß man die Benzoesäuren der allgemeinen Formel II

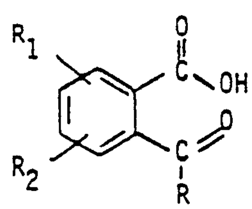

(II)

in der R, $R_1$ und $R_2$ die in Anspruch 1 angegebenen Bedeutungen besitzen, mit $SOCl_2$ zur Bildung von Isobenzofuranonen der allgemeinen Formel III

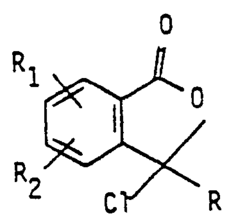

(III)

behandelt, welche man mit der Verbindung der allgemeinen Formel IV

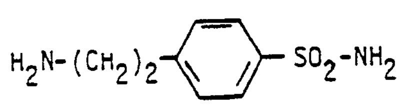

(IV)

zu einem 3-Hydroxyisoindolon der allgemeinen Formel V

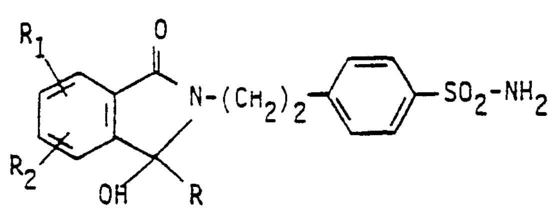

(V)

in der R, $R_1$ und $R_2$ die in Anspruch 1 angegebenen Bedeutungen besitzen, kondensiert, welche Verbindungen der allgemeinen Formel V man in die Isoindolone der allgemeinen Formel VI

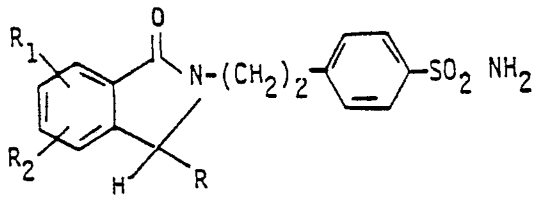

(VI)

in der R, $R_1$ und $R_2$ die in Anspruch 1 angegebenen Bedeutungen besitzen, unwandelt, und man diese Indolone der Formel (VI) mit einer Verbindung der Formel VII

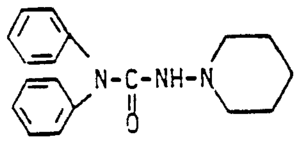

(VII)

kondensiert.